(19) Europäisches Patentamt

European Patent Office

Office européen des brevets

(11) Veröffentlichungsnummer: **0 367 902 B1**

# (12) EUROPÄISCHE PATENTSCHRIFT

(45) Veröffentlichungstag der Patentschrift: **02.03.94**

(21) Anmeldenummer: **89111820.0**

(22) Anmeldetag: **29.06.89**

(51) Int. Cl.⁵: **C07D 475/04**

---

(54) Verfahren zur Trennung von Folinsäure.

---

(30) Priorität: **11.11.88 CH 4182/88**

(43) Veröffentlichungstag der Anmeldung:
**16.05.90 Patentblatt 90/20**

(45) Bekanntmachung des Hinweises auf die
Patenterteilung:
**02.03.94 Patentblatt 94/09**

(84) Benannte Vertragsstaaten:
**AT BE CH DE ES FR GB GR IT LI LU NL SE**

(56) Entgegenhaltungen:
**EP-A- 0 266 042**
**WO-A-88/08844**
**US-A- 2 688 018**

(73) Patentinhaber: **EPROVA Aktiengesellschaft**
**Im Laternenacker 5**
**CH-8200 Schaffhausen(CH)**

(72) Erfinder: **Müller, Hans Rudolf**
**Beckenwäldli 18**
**CH-8207 Schaffhausen(CH)**
Erfinder: **Ulmann, Martin**
**Steigstrasse 36**
**CH-8447 Dachsen(CH)**
Erfinder: **Conti, Josef**
**Winkelriedstrasse 22**
**CH-8203 Schaffhausen(CH)**
Erfinder: **Mürdel, Günter**
**vor Hegin**
**D-7708 Tengen-Büsslingen(DE)**

---

## Beschreibung

Die Erfindung betrifft ein Verfahren zur Trennung von N 5-Formyl-(6R,S)-5,6,7,8-tetrahydrofolsäure -5-CHO-(6R,S)-THF - kurz Folinsäure genannt, und Isolierung von N 5-Formyl-(6S)-5,6,7,8-tetrahydrofolsäure -5-CHO-(6S)-THF-, dem biochemisch aktiven Citrovorum-Faktor ( = Wachstumsfaktor für Leuconostoc citrovorum).

Die Folinsäure enthält 2 asymmetrische Zentren. Dabei liegt aufgrund der Synthese der Folinsäure aus Folsäure, der N-(Pteroyl)-L-glutaminsäure, das im Glutaminsäure-Rest enthaltene optisch aktive C-Atom in der L-Form vor, während das durch Hydrierung der Doppelbindung in 5,6-Stellung des Pteroyl-Restes entstandene optisch aktive C-Atom in Position 6 in der racemischen, der (6R,S)-Form, vorliegt. Synthetische Folinsäure ( = Leucovorin) besteht demnach aus einer 1:1-Mischung von zwei Diastereomeren.

In natürlichen Vorkommen, z.B. in der Leber, findet man die Folinsäure nur in der (6S)-Form als 5-CHO-(6S)-THF. 5-CHO-(6R,S)-THF (Folinsäure), wird in Form ihres Calciumsalzes (Leucovorin Calcium) als Arzneimittel verwendet zur Behandlung von megaloblastischer Folsäuremangel-Anämie, als Antidot zur Verstärkung der Verträglichkeit von Folsäure-Antagonisten speziell von Aminopterin, Methotrexat und Fluoruracil in der Krebstherapie ("leucovorin rescue") und der Behandlung von Autoimmunkrankheiten wie Psoriasis und rheumatischer Arthritis sowie zur Verstärkung der Verträglichkeit von bestimmten Antiparasitika, etwa Trimethoprim-Sulfamethoxazol, in der Chemotherapie.

Nach Verabreichung von 5-CHO-(6R,S)-THF wird der (6S)-Anteil dieser Diasteromeren-Mischung rasch in 5-Me-(6S)-THF umgewandelt, während der (6R)-Anteil nicht metabolisiert und langsam durch den Harn ausgeschieden wird: J.A. Straw et al., Cancer Research 44, 3114-3119 (1984).

F.M. Sirotnak et al., Biochemical Pharmacology 28, 2993-97 (1979) haben gefunden, dass zur Unterdrückung der Zellwachstumshemmung durch Methotrexat in Kulturen von L 1210 das unnatürliche Diastereomere der Folinsäure [5-CHO-(6R)-THF] 100-mal und die chemisch-synthetische Diastereomerenmischung [5-CHO-(6R,S)-THF] 2-mal weniger wirksam ist als das natürliche Diastereomere [5-CHO-(6S)-THF].

C. Temple et al., Cancer Treatment Reports 65, 1117-9 (1981) haben diese in vitro bestimmten Resultate in vivo bestätigt und gefunden, dass das natürliche Diastereomere bezüglich der Reduktion der Toxizität von Methotrexat mehr als 2-mal wirksamer ist als die Mischung der Diastereomeren [Leucovorin].

Diese Autoren haben sogar eine mögliche schädliche Wirkung des unnatürlichen Diastereomeren vermutet.

5-CHO-(6R)-THF hemmt nämlich einige für den $C_1$-Transfer verantwortliche Enzyme und damit die biochemische Wirkung der Tetrahydrofolate: R.P. Leary et al., Biochem. Biophys. Resp Commun. 56, 484 (1973); V.F. Scott et al., ibid. 14, 523 (1964); G.K. Smith et al., Biochemistry, 20, 4034 (1981). Die Verwendung von (6S)-Tetrahydrofolaten anstelle von (6R,S)-Tetrahydrofolaten müsste daher neben doppelter Wirksamkeit auch qualitative therapeutische Vorteile aufweisen.

Es besteht daher das Bedürfnis, die bisher verwendete 1:1-Mischung der Diastereomeren durch den natürlichen Wirkstoff [5-CHO-(6S)-THF] zu ersetzen.

Es sind mehrfach Anstrengungen zur Trennung von 5-CHO-(6R,S)-THF und zur asymmetrischen Synthese von 5-CHO-(6S)-THF unternommen worden.

D. Cosulich et al., J. Amer. chem. Soc. 74, 4215-16 (1952), US-PS 2.688.018 (31.08.1954) haben beispielsweise versucht, die Trennung durch fraktionierte Kristallisation eines Erdalkalisalzes, z.B. des Calcium- oder Strontiumsalzes der 5-CHO-(6R,S)-THF aus wässeriger Lösung zu bewerkstelligen [siehe auch J.C. Fontecilla-Camps et al., J. Amer. chem. Soc. 101, 6114 (1979)].

Unter den von D. Cosulich et al. offenbarten Bedingungen lässt sich jedoch die gewünschte Trennung nicht realisieren. Bei der Kristallisation z.B. des Calciumsalzes von 5-CHO-(6R,S)-THF aus Wasser bei pH 7-8 wird immer wieder die 6R,S-Form erhalten, wie sich mittels chromatographischer Analyse an einer chiralen HPLC-Säule sowie anhand der optischen Drehung quantitativ nachweisen lässt. Dabei ist es unerheblich, ob man rohes oder reines Calciumsalz von 5-CHO-(6R,S)-THF zur Kristallisation einsetzt; stets wird die (6R,S)-Form zurückerhalten. Eine Trennung und Anreicherung der (6S)-Form kann auch nicht erreicht werden, wenn man die übersättigte wässerige Lösung eines Erdalkalisalzes von 5-CHO-(6R,S)-THF mit authentischem Erdalkalisalz von 5-CHO-(6S)-THF impft.

Die Trennung der Diastereomeren-Paare wurde auch mittels Chromatographie versucht: J. Feeney et al., Biochemistry 20, 1837 (1981). Ausserdem wurden die (6S)-Isomeren hergestellt durch stereospezifische Reduktion von Dihydrofolaten in Gegenwart von Dihydrofolat-Reduktase: L. Rees et al., Tetrahedron 42, 117 (1986).

L. Rees et al., J. Chem. Soc., Chem. Commun. 1987, 470, EP-A2-0 266 042 haben ein Trennverfahren für (6R,S)-THF beschrieben, mit dessen Hilfe 5-CHO-(6S)-THF und 5-CHO-(6R)-THF in geringen Mengen erzeugt werden konnten. Das Verfahren besteht darin, dass man (6R,S)-THF mit (-)-Menthyl-chloroformiat zu den diastereomeren 5-(-)-Menthyloxycarbonyl-tetrahydrofolsäuren umsetzt, diese durch wiederholte Behandlung mit n-Butanol trennt, die erhaltenen Diastereomeren mit einer gesättigten Lösung von Bromwasserstoff in einer Mischung aus Ameisensäure und Essigsäure erwärmt, wobei nach Hydrolyse 5-Formyl-(6S)- und (6R)-THF gebildet werden, und schliesslich diese als Calciumsalze isoliert.

Dieses Verfahren ist umständlich und schwierig und benötigt zur Herstellung des chiralen Reagens hochgiftiges Phosgen. Zudem ist das Ausgangsmaterial (6R,S)-THF sehr unbeständig. Bei der Abspaltung der chiralen Hilfsgruppe mit HBr in AcOH bei > 50°C wird ein Teil der Glutaminsäure abgespalten und es entstehen Nebenprodukte, die sich nur schwierig abtrennen lassen. Die nach einem solchen Verfahren erzeugte (6S)-Folinsäure wäre so teuer, dass deren Anwendung anstelle der (R,S)-Tetrahydrofolate kaum in Betracht kommt.

Bis heute ist also kein brauchbares Verfahren zur Gewinnung von (6S)-Tetrahydrolaten bekannt geworden.

Es bestand somit nach wie vor die Aufgabe, ein einfaches, industriell anwendbares Verfahren zur Herstellung von 5-CHO-(6S)-THF zu finden.

Es wurde nun überraschend gefunden, dass aus wässerigen Lösungen von Salzen der (6R,S)-Folinsäure [5-CHO-(6R,S)-THF] nach Zusatz von wasserlöslichen Alkali-, Ammonium- oder Erdalkalisalzen anorganischer oder organischer Säuren sich entsprechendes Folinat ausscheidet, das stark überwiegend aus dem unnatürlichen diastereomeren (6R)-Folinat [5-CHO-(6R)-THF] besteht. Dieses lässt sich durch Filtration abtrennen. Aus dem Filtrat, welches vorwiegend das gewünschte, natürliche (6S)-Folinat enthält, lässt sich durch Zusatz eines mit Wasser mischbaren organischen Lösungsmittels wie etwa Ethanol oder Aceton und/oder eines wasserlöslichen Erdalkalisalzes das (6S)-Folinat isolieren. Dieses kann anschliessend durch Umkristallisation von Resten des (6R)-Folinates befreit werden.

Unter "Ammoniumsalz" werden hier und im folgenden unsubstituierte oder 1- bis 4-fach substituierte Ammoniumsalze verstanden, einschliesslich der Pyrrolidinium-, Piperidinium- und Morpholinium-salze. Als Substituenten kommen insbesondere in Frage Alkyl- oder Hydroxyalkylgruppen mit 1 bis 4 C-Atomen oder Benzyl.

Die Trennung von Erdalkalisalzen von (6R,S)-Folinaten funktioniert nach diesem Verfahren nur bei annähernd neutralem pH. Aus alkalischem Milieu (pH > 8) wird, in Übereinstimmung mit dem in der PCT/EP-Patentanmeldung 88/00341 (publiziert am 17. Nov. 1988 als WO 88/08844) beschriebenen Verfahren, zunächst vorwiegend aus Erdalkali-(6S)-Folinat bestehendes Produkt ausgeschieden. Bei der Trennung von Alkali-(6R,S)-Folinaten scheidet sich nach Zusatz von Alkalihalogeniden sowohl in annähernd neutralem als auch aus alkalischem Milieu zunächst das (6R)-Folinat aus.

Es ist nicht erforderlich und im allgemeinen auch nicht zweckmässig, dass die Kationen der zu trennenden Mischung der Diastereomeren und diejenige der Alkali-, Ammonium- bzw. Erdalkalisalze einer anorganischen oder niedrigen organischen Säure übereinstimmen.

Wenn man etwa eine Lösung eines Erdalkalisalzes von (6R,S)-Folinat bei annähernd neutralem pH mit einem wasserlöslichen Alkalihalogenid, beispielsweise mit Natriumiodid, versetzt, so scheidet sich zunächst das entsprechende Erdalkali-(6R)-Folinat aus.

Ähnliches tritt ein, wenn man eine Lösung eines Alkali-(6R,S)-Folinates bei annähernd neutralem pH mit einem wasserlöslichen Erdalkalisalz versetzt. Zunächst scheidet sich das entsprechende Erdalkali-(6R)-Folinat aus.

Wenn man eine Lösung von Alkali-(6R,S)-Folinat mit löslichen Alkalisalzen anorganischer Säuren versetzt, so scheidet sich, mitunter erst nach Zusatz eines organischen Lösungsmittels wie Methanol, Ethanol, Isopropanol oder Aceton, zunächst das entsprechende Alkali-(6R)-Folinat aus.

Aus der Mutterlauge lässt sich durch Zusatz von (mehr) organischem Lösungsmittel das Alkali-(6S)-Folinat zur Ausscheidung veranlassen. Man kann aber aus der Mutterlauge auch durch Zusatz von wasserlöslichen Erdalkalisalzen das entsprechende Erdalkali-(6S)-Folinat zur Ausscheidung bringen. Dabei schadet es nicht, wenn das pH der Lösung auf ≧ 8 ansteigt.

Die erforderliche Menge an wasserlöslichen Alkali- oder Ammoniumsalzen anorganischer oder organischer Säuren ist verhältnismässig hoch. Sie beträgt etwa die 0,5- bis 10-fache, vorzugsweise 1- bis 5-fache, Gewichtsmenge bezogen auf das Gewicht des eingesetzten (6R,S)-Folinates. Wasserlösliche Erdalkalisalze werden in weit geringeren Mengen benötigt.

Bei Einhaltung optimaler Bedingungen, die durch Versuchsreihen zu ermitteln sind, scheidet sich das (6R)-Folinat in optisch praktisch reiner Form und mit hoher Ausbeute aus. Unter solchen Verhältnissen enthält die Mutterlauge jeweils nur noch wenig (6R)-Folinat, und dementsprechend weist das anschliessend beispielsweise durch Zusatz von organischem Lösungsmittel oder einem Erdalkalisalz zur Ausscheidung gebrachte (6S)-Folinat einen hohen (6S)-Anteil von 70 bis über 95% auf. Derartiges mit der (6S)-Form stark angereichertes Folinat lässt sich durch Umkristallisation von begleitendem (6R)-Folinat vollständig befreien. Wenn der Gehalt an (6S)-Folinat jedoch nur etwa 60% oder weniger beträgt, wird durch Umkristallisation das Racemat, die (6R,S)-Form, zurückgebildet und die überschüssige (6S)-Form bleibt in Lösung.

Das Verfahren ist einfach und bei optimaler Prozessführung auch sehr ergiebig.

Gegenstand der Erfindung ist ein Verfahren zur Trennung von (6R,S)-Folinaten, dadurch gekennzeichnet, dass man eine wässerige Lösung eines Salzes der (6R,S)-Folinsäure mit einem wasserlöslichen Alkali-, Ammonium- oder Erdalkalisalz einer anorganischen oder organischen Säure versetzt, das sich ausscheidende (6R)-Folinat abfiltriert und aus dem Filtrat das (6S)-Folinat isoliert.

Die Isolierung des (6S)-Folinates erfolgt dadurch, dass das (6S)-Folinat aus dem Filtrat durch Zusatz eines mit Wasser mischbaren organischen Lösungsmittels und/oder vorzugsweise eines wasserlöslichen Erdalkalisalzes ausgeschieden, abfiltriert und gegebenenfalls umkristallisiert wird.

Bevorzugt wird die Abtrennung von Erdalkalisalzen der (6R)-Folinsäure bei annähernd neutralem pH durchgeführt.

Als Ausgangsmaterial besonders geeignete Salze von (6R,S)-Folinsäure sind das Natrium-, Kalium-, Calcium-, Magnesium-, Strontium- oder Barium-Salz. Das Natrium- und insbesondere das Calcium- und Magnesium-Salz werden bevorzugt, weil diese nach erfolgter Trennung, Isolierung und Reinigung direkt als Arzneimittel verwendbar sind.

Bei den anorganischen oder organischen Säuren, die den entsprechenden Salzen zugrundeliegen, handelt es sich vorzugsweise um starke Säuren, wobei die organischen Säuren bevorzugt 1 bis 4 C-Atome enthalten.
Als anorganische Säuren seien genannt: Iodwasserstoffsäure, Bromwasserstoffsäure, Chlorwasserstoffsäure, Salpetersäure und als organische Säuren: Ameisensäure, Milchsäure, Citronensäure, Methansulfonsäure.

Als wasserlösliche Alkali-, Ammonium- oder Erdalkalisalze haben sich die Halogenide, insbesondere die Iodide und Bromide, sehr gut bewährt.
Im einzelnen sind bevorzugt: Natriumiodid, Natriumbromid, Natriumchlorid, Kaliumiodid, Kaliumbromid, Kaliumchlorid, Ammoniumiodid, Ammoniumbromid, Ammoniumchlorid, Calciumiodid, Calciumbromid, Calciumchlorid, Magnesiumbromid, Magnesiumchlorid, Strontiumbromid, Strontiumchlorid, Bariumiodid, Bariumbromid, Bariumchlorid.

Ein Zusatz eines wasserlöslichen Erdalkalisalzes vorzugsweise von Calciumiodid, Calciumbromid, Calciumchlorid, Magnesiumbromid oder Magnesiumchlorid erfolgt zweckmässig zu einer Lösung eines Alkali-(6R,S)-Folinates.

Die erfindungsgemässe Trennung gelingt aber auch durch Zusatz anderer wasserlöslicher Alkali-, Ammonium- und Erdalkalisalze wie beispielsweise Natriumnitrat, Kaliumnitrat, Ammoniumnitrat, Natriumformiat, Natriumlactat, Natriumcitrat, Calciumlactat oder Natriummethansulfonat.

Als mit Wasser mischbare organische Lösungsmittel zur Isolierung der (6S)-Folinate eignen sich niedrige Alkohole wie Methanol, Ethanol, n-Propanol, Isopropanol, Glykolether wie 2-Methoxyethanol, 2-Ethoxyethanol, 2-Butyloxyethanol, 1-Methoxy-2-propanol, 1,2-Dimethoxyethan, 1,2-Diethoxyethan ferner Dioxan, Tetrahydrofuran, niedrige Ketone wie Aceton, Methylethylketon, Methoxyaceton, 1,3-Dimethoxyaceton sowie Acetonitril. Bevorzugt werden niedrige Alkohole oder Ketone, insbesondere Methanol, Ethanol, Isopropanol und Aceton.

Als wasserlösliche Erdalkalisalze zur Ausscheidung entsprechender (6S)-Folinate eignen sich Erdalkalihalogenide wie z.B. Calciumchlorid, Calciumbromid, Calciumiodid, Magnesiumchlorid, Magnesiumbromid, Strontiumchlorid, Strontiumbromid oder ein Bariumhalogenid. Bevorzugt für diesen Zweck werden Calciumchlorid und Magnesiumchlorid.

Die Erfindung ist demnach weiter dadurch gekennzeichnet, dass man als Alkali- bzw. Erdalkalisalz von (6R,S)-Folinsäure vorzugsweise das Natrium-, Calcium- oder Magnesiumsalz, als wasserlösliches Salz einer anorganischen Säure vorzugsweise ein Natrium-, Kalium- oder Ammoniumhalogenid, insbesondere Natriumiodid, Natriumbromid, Natriumchlorid, Kaliumiodid, Kaliumbromid, Kaliumchlorid, Ammoniumiodid, Ammoniumbromid und/oder Ammoniumchlorid und zur Ausscheidung des entsprechenden (6S)-Folinates als organisches Lösungsmittel bevorzugt einen niedrigen Alkohol oder ein niedriges Keton und/oder als wasserlösliches Erdalkalisalz ein Calcium- oder Magnesiumhalogenid verwendet.

Beispiele zur Ilustrierung der Erfindung:

**Beispiel 1**

**Trennung von Calcium-(6R,S)-Folinat und Isolierung von Calcium-(6R)-Folinat sowie von Calcium-(6S)-Folinat.**

50 g reines Calcium-(6R,S)-Folinat werden in 500 ml Wasser gelöst und mit 100 g Natriumiodid versetzt. Das pH der Lösung wird auf 7,0 eingestellt. Die Lösung wird nun unter Rühren auf 1°C abgekühlt. Das pH der Lösung steigt auf etwa 7,7. Nach 15-20 Std. wird das ausgeschiedene Produkt abfiltriert, mit konzentrierter wässeriger Natriumiodid-Lösung und danach mit Ethanol gewaschen.
Man erhält Calcium-(6R)-Folinat mit (6R)-Gehalt von 98,1%; bestimmt mittels HPLC unter Verwendung einer chiralen Säule (Resolvosil-BSA-7). Nach Umkristallisation aus Wasser in Gegenwart von Calciumchlorid erhält man reines Calcium-(6R)-Folinat mit einem (6R)-Gehalt von 100,0%.
Spezifische Drehung $[\alpha]_D^{20} = + 43,7°$ (bezogen

auf wasserfreies Ca-Salz).

Löslichkeit in Wasser: bei 20°C: 2,0 g/100 ml

Das Filtrat wird mit Ethanol versetzt und unter Rühren auf 3°C abgekühlt. Nach etwa 15-20 Std. wird das ausgeschiedene Produkt abfiltriert und mit Ethanol gewaschen. Man erhält Calcium Folinat, enthaltend 84% Calcium-(6S)-Folinat und 16% Calcium-(6R)-Folinat.

Durch Umkristallisieren aus Wasser in Gegenwart von Calciumchlorid erhält man daraus Calcium-(6S)-Folinat mit einem (6S)-Gehalt von 97,6%. Nach einer weiteren Umkristallisation beträgt der (6S)-Gehalt ≧ 99,5%.

Spezifische Drehung: $[\alpha]_D^{20}$ = - 15°C (bezogen auf wasserfreies Ca-Salz).

**Beispiel 2**

**Trennung von (6R,S)-Folinsäure und Isolierung von Natrium-(6R)-Folinat und Calcium-(6S)-Folinat.**

25 g (6R,S)-Folinsäure werden in 70 ml Wasser aufgeschlämmt und durch Zusatz von 49 ml 2N Natronlauge in Lösung gebracht. Die Lösung wird mit 40 g Natriumchlorid und 140 g Natriumiodid versetzt. Es bildet sich eine gallertige Ausscheidung. Diese wird nach einigen Stunden abfiltriert und mit Methanol ausgewaschen.

Man erhält optisch reines Natrium-(6R)-Folinat mit einem (6R)-Gehalt von 99%.

Die Mutterlauge wird mit 25 g Calciumchlorid versetzt und auf pH 8,5 gestellt, worauf sich allmählich Calcium-(6S)-Folinat mit einem (6S)-Gehalt von 78% ausscheidet. Dieses rohe Calcium-(6S)-Folinat wird bei 30-35°C in Wasser gelöst, mit Aktiv-Kohle und einem Filterhilfsmittel (Solkafloc$^{(R)}$) versetzt, filtriert, auf eine Konzentration von 10% eingedampft und bei 5 bis 1°C kristallisieren gelassen.

Man erhält Calcium-(6S)-Folinat mit einem (6S)-Gehalt von annähernd 98%.

**Beispiel 3**

**Trennung von (6R,S)-Folinsäure und Isolierung von Natrium-(6R)-Folinat und Magnesium-(6S)-Folinat.**

25 g (6R,S)-Folinsäure werden in 75 ml Wasser aufgeschlämmt und durch Zusatz von 5 N wässerigem Natriumhydroxid in Lösung gebracht. Die schwach alkalische Lösung wird unter Rühren mit 75 g Natriumbromid und danach mit 200 ml Methanol versetzt. Nach 2 Wochen kristallisiert allmählich Natrium-(6R)-Folinat aus.

Dieses Produkt wird abfiltriert, mit Methanol gewaschen und getrocknet.

Man erhält optisch praktisch reines Natrium-(6R)-Folinat mit einem (6R)-Gehalt von 98%.

Spezifische Drehung $[\alpha]_D^{24}$ = + 39,5° (bezogen auf wasserfreies Na-Salz, c = 1% in Wasser)

Die Mutterlauge wird von Methanol befreit, auf pH 9,8 gestellt und mit 25 g Magnesiumchlorid versetzt und langsam gerührt. Nach einiger Zeit kristallisiert Magnesium-(6S)-Folinat allmählich aus. Es wird nach etwa 100 Std. abfiltriert, mit Ethanol ausgewaschen und getrocknet. (6S)-Gehalt 85%. Durch Umkristallisation in Gegenwart von Magnesiumchlorid erhält man optisch reines Magnesium-(6S)-Folinat.

**Beispiel 4**

**Trennung von Natrium-(6R,S)-Folinat und Isolierung der beiden Diastereomeren.**

25 g (6R,S)-Folinsäure werden in 75 ml Wasser durch Zusatz von 5 N wässerigem Natriumhydroxid in Lösung gebracht bei 20°C mit 140 g Natriumiodid versetzt und unter Rühren auf 5°C abgekühlt.

Nach Stehen über Nacht wird das ausgeschiedene Natrium-(6R)-Folinat abfiltriert und mit Aceton gewaschen. Das erhaltene Produkt weist einen (6R)-Anteil von 82,9% auf.

Die vereinigten Mutterlaugen werden mit 600 ml Aceton versetzt und bei 0°C gerührt. Die entstandene Ausscheidung wird abfiltriert. Sie besteht vorwiegend aus Natrium-(6S)-Folinat mit einem (6S)-Anteil von 71%.

**Beispiel 5**

**Trennung von Calcium-(6R,S)-Folinat durch Zusatz von Ammoniumiodid in annähernd neutralem Milieu.**

50 g Calcium-(6R,S)-Folinat und 100 g Ammoniumiodid werden in 500 ml Wasser bei 36°C gelöst. Die Lösung weist ein pH von 7,7 auf. Man lässt nun langsam unter Rühren auf Raumtemperatur abkühlen, kühlt unter stetigem Rühren weiter auf 5°C und schliesslich auf 0-2°C ab, impft die Lösung durch Zugabe von einigen Milligramm reinem Calcium-(6R)-Folinat, worauf Kristallisation einsetzt.

Das dabei auskristallisierte, vorwiegend aus Calcium-(6R)-Folinat bestehende, Produkt wird abfiltriert, mit Ethanol gewaschen und getrocknet. Gehalt an (6R)-Folinat: 98%.

Das Filtrat wird mit den Wasch-Lösungen vereinigt und unter Rühren zusätzlich mit 1 lt Ethanol versetzt.

Die sich allmählich ausscheidende, zur Hauptsache aus Calcium-(6S)-Folinat bestehende Fraktion wird abfiltriert, mit Ethanol gewaschen und getrocknet. Der Gehalt an (6S)-Folinat beträgt 96%.

Erhaltenes rohes Calcium-(6R)-Folinat und Calcium-(6S)-Folinat werden jeweils aus wenig Wasser unter Zusatz von Aktivkohle und Filterhilfsmittel umkristallisiert, wodurch der Gehalt an (6R)- bzw. (6S)-Folinat auf 99,5-99,7% ansteigt.

**Beispiel 6**

**Trennung von Calcium-(6R,S)-Folinat durch Zusatz von Natriumbromid in annähernd neutralem Milieu und anschliessende Isolierung von Calcium-(6S)-Folinat durch Zusatz von CaCl$_2$.**

50 g Calcium-(6R,S)-Folinat in 500 ml Wasser werden mit 75 g Natriumbromid versetzt. Das pH der Lösung beträgt 7,4. Man kühlt unter Rühren von 35°C auf 1-3°C ab, worauf langsame Kristallisation einsetzt.

Das Kristallisat wird abfiltriert und mit Methanol gewaschen. Erhaltenes aus Calcium-(6R)-Folinat bestehendes Kristallisat weist einen (6R)-Gehalt von 93% auf.

Das Filtrat wird mit 100 g Calciumchlorid versetzt, worauf sich Calcium-(6S)-Folinat ausscheidet. Es wird abfiltriert und mit Ethanol gewaschen. Erhaltenes Ca-(6S)-Folinat hat einen (6S)-Gehalt von 84%. Durch Umkristallisation aus wenig Wasser in Gegenwart von Calciumchlorid erhält man in guter Ausbeute reines Calcium-(6S)-Folinat mit einem (6S)-Gehalt von 99,6%.

**Beispiel 7**

**Trennung von Calcium-(6R,S)-Folinat in annähernd neutralem Milieu durch Zusatz von Ammoniumbromid.**

50 g Calcium-(6R,S)-Folinat in 500 ml Wasser werden mit 70 g Ammoniumbromid versetzt. Das pH der Lösung beträgt 6,5. Die erhaltene Lösung wird unter Rühren auf 0°C abgekühlt und mit authentischem Ca-(6R)-Folinat geimpft. Allmählich setzt Kristallisation ein. Nachdem der Ca-(6S)-Gehalt der überstehenden Lösung nicht mehr zunimmt, d.h. sich keine weiteren Mengen von Ca-(6R)-Folinat ausscheiden, wird abfiltriert und mit Methanol und Ethanol gewaschen.

Erhaltenes Ca-(6R)-Folinat weist einen (6R)-Gehalt von 93,8% auf.

Das Filtrat wird mit 100 g Calciumchlorid versetzt und auf pH 7 eingestellt, worauf sich allmählich Ca-(6S)-Folinat ausscheidet. Erhaltenes Produkt wird abfiltriert und mit Ethanol gewaschen. Der Gehalt an Ca-(6S)-Folinat beträgt 98,7%. Durch Umkristallisation in Gegenwart von Calciumchlorid wird daraus reines Ca-(6S)-Folinat gewonnen.

**Beispiel 8**

**Trennung von Calcium-(6R,S)-Folinat durch Zusatz von Kaliumiodid**

50 g Calcium-(6R,S)-Folinat werden mit 500 ml Wasser und 110 g Kaliumiodid versetzt und bei 55°C in Lösung gebracht. Die erhaltene Lösung (pH 7,1) wird unter Rühren stufenweise auf 0°C abgekühlt und mit authentischem Ca-(6R)-Folinat geimpft. Nach Stehenlassen über Nacht wird das ausgeschiedene Kristallisat abfiltriert, mit wenig Kaliumiodid-Lösung und mit Ethanol gewaschen. Das so erhaltene Ca-(6R)-Folinat ist ziemlich rein. Der Gehalt an (6R)-Diastereomerem beträgt 99,1%.

Das Filtrat wird bei 60°C mit 100 g Calciumchlorid in 50 ml Wasser versetzt, durch Zusatz von wenig Natriumhydroxid auf pH 7,3 eingestellt und stufenweise auf 15°C abgekühlt, wobei Calcium-(6S)-Folinat auskristallisiert.

Nach 3 Tagen wird das Kristallisat abfiltriert, mit kalter Calciumchlorid-Lösung und mit Ethanol gewaschen. Das in hoher Ausbeute erhaltene rohe Calcium-(6S)-Folinat weist einen (6S)-Gehalt von 97,3% auf.

**Beispiel 9**

**Trennung von Calcium-(6R,S)-Folinat durch Zusatz von Natriumchlorid.**

50 g reines Calcium-(6R,S)-Folinat werden bei 60°C in 320 ml Wasser gelöst, mit 35 g Natriumchlorid versetzt und unter Rühren allmählich auf 0°C abgekühlt und dabei mit authentischem Ca-(6R)-Folinat geimpft. Das langsam sich ausscheidende Kristallisat wird abfiltriert. Es besteht aus rohem Ca-(6R)-Folinat mit einem (6R)-Gehalt von 82,5%.

Aus dem Filtrat wird nach Zusatz von Calciumchlorid und Ethanol Ca-(6S)-Folinat erhalten.

Wenn man in gleicher Weise verfährt, aber die Menge des zugesetzten Natriumchlorids auf 17,5 g halbiert, so erhält man rohes Ca-(6R)-Folinat mit einem (6R)-Anteil von nur knapp 67%. Etwa dasselbe Resultat wird erhalten, wenn man zwar 35 g Natriumchlorid zusetzt, aber anstelle von Ca-(6R)-Folinat Ca-(6R,S)-Folinat zum Impfen verwendet.

**Beispiel 10**

**Trennung von 50 g Calcium-(6R,S)-Folinat in 500 ml Wasser durch Zusatz von 35 g Ammoniumchlorid analog Beispiel 7.**

Man erhält Ca-(6R)-Folinat mit einem (6R)-Gehalt von 92,7% und, nach Zusatz von 100 g Calciumchlorid zur Mutterlauge, Ca-(6S)-Folinat mit einem (6S)-Gehalt von 97%.

**Beispiel 11**

**Trennung von 50 g Calcium-(6R,S)-Folinat durch Zusatz von 100 g Tetramethylammoniumbromid in 500 ml Wasser analog Beispiel 7.**

Erhalten werden Ca-(6R)-Folinat mit einem (6R)-Gehalt von 89,1% und Ca-(6S)-Folinat mit einem (6S)-Gehalt von 97,4%.

**Beispiel 12**

**Trennung von 50 g Calcium-(6R,S)-Folinat in 400 ml Wasser durch Zusatz von ca. 125 g Diethanolamin-hydrobromid in 100 ml Wasser.**

Man erhält bei der analog Beispiel 7 durchgeführten Trennung:
Ca-(6R)-Folinat mit einem (6R)-Gehalt von 99,1% und
Ca-(6S)-Folinat mit einem (6S)-Gehalt von 80%.

**Beispiel 13**

**Trennung von aus 5,10-Methenyl-tetrahydrofolsäure [5,10-CH-(6R,S)-THF = Anhydroleucovorin] in situ erhaltenem Calcium-(6R,S)-Folinat mit Ammoniumbromid oder Ammoniumchlorid.**

12,6 g Calciumhydroxid werden in 900 ml Wasser bei 90°C gelöst. Die Lösung wird unter Rühren innert weniger Minuten mit 110 g Anhydroleucovorinbromid-hydrobromid ([5,10-CH-(6R,S)-THF]$^{(+)}$Br$^{(-)}$•HBr) und gleichzeitig mit 25%igem wässrigem Ammoniak versetzt, um das pH der Reaktionslösung stets auf 6 zu halten. Die erhaltene Lösung wird noch 3-4 Stunden bei pH 6 am Rückfluss gekocht, wobei sich Ca-(6R,S)-Folinat (5-CHO-THF) bildet. Die Reaktionslösung wird durch Behandlung mit Aktivkohle und Filterhilfsmittel geklärt mit 100 g Ammoniumbromid versetzt, auf pH 7 eingestellt, abgekühlt und mit authentischem Ca-(6R)-Folinat geimpft, wonach sich allmählich der Grossteil des vorhandenen Ca-(6R)-Folinates ausscheidet. Das Kristallisat wird abfiltriert und gewaschen. Es weist einen (6R)-Gehalt von 98,3% auf.
Das Filtrat wird mit 200 g Calciumchlorid in 100 g Wasser versetzt, auf pH 7 eingstellt und mit authentischem Ca-(6S)-Folinat geimpft, worauf das vorhandene Ca-(6S)-Folinat auskristallisiert. Das Kristallisat wird abfiltriert. Es weist einen (6S)-Gehalt von 92% auf.

Im vorstehenden Beispiel können Anhydroleucovorinbromid-hydrobromid auch durch die äquivalente Menge Anhydroleucovorinchlorid-hydrochlorid und das Ammoniumbromid durch dieselbe Menge Ammoniumchlorid ersetzt werden. Es wird dabei ein ähnliches Resultat erzielt.

Auf ähnliche Weise, wie in den vorstehenden Beispielen beschrieben, werden folgende analoge Trennungen durchgeführt:

**Beispiel 14**

Trennung von Magnesium-(6R,S)-Folinat in seine Komponenten durch Zusatz von Ammoniumbromid in annähernd neutralem Milieu.

**Beispiel 15**

Trennung von Strontium-(6R,S)-Folinat durch Zusatz von Natriumiodid, Abtrennung von Sr-(6R)-Folinat und anschliessende Ausscheidung von Sr-(6S)-Folinat durch Zusatz von 2-Methoxyethanol und Strontiumchlorid.

**Beispiel 16**

Trennung von Barium-(6R,S)-Folinat und Isolierung von Ba-(6R)-Folinat und von Ba-(6S)-Folinat zunächst durch Zusatz von Bariumiodid und danach von 1,2-Dimethoxy-ethan.

**Beispiel 17**

Trennung von Natrium-(6R,S)-Folinat durch Zusatz von Magnesiumbromid, Abtrennung von Magnesium-(6R)-Folinat, Zusatz von Calciumchlorid zum Filtrat und Isolierung von Calcium-(6S)-Folinat.

**Beispiel 18**

Trennung von Calcium-(6R,S)-Folinat durch Zusatz von Magnesiumbromid, Abtrennung von ausgeschiedenem (6R)-Folinat, Zusatz von Calciumchlorid zum Filtrat und Isolierung von dabei ausgeschiedenem Calcium-(6S)-Folinat.

**Beispiel 19**

Trennung von Ca-(6R,S)-Folinat durch Zusatz von Tetraethylammoniumbromid.

**Beispiel 20**

Trennung von Ca-(6R,S)-Folinat durch Zusatz von Tetrabutylammoniumbromid.

**Beispiel 21**

Trennung von Ca-(6R,S)-Folinat durch Zusatz von Triethylamin-hydrochlorid.

**Beispiel 22**

Trennung von Mg-(6R,S)-Folinat durch Zusatz von Diethanolamin-hydrochlorid.

**Beispiel 23**

Trennung von Ca-(6R,S)-Folinat durch Zusatz von Benzylamin-hydroiodid.

**Beispiel 24**

Trennung von Ca-(6R,S)-Folinat durch Zusatz von Morpholin-hydrobromid.

**Patentansprüche**

1. Verfahren zur Trennung von (6R,S)-Folinaten, dadurch gekennzeichnet, dass man eine wässerige Lösung eines Salzes der (6R,S)-Folinsäure mit einem wasserlöslichen Alkali-, Ammonium- oder Erdalkalisalz einer anorganischen oder organischen Säure versetzt, das sich ausscheidende (6R)-Folinat abfiltriert und aus dem Filtrat das (6S)-Folinat isoliert.

2. Verfahren nach Anspruch 1, dadurch gekennzeichnet, dass das (6S)-Folinat aus dem Filtrat durch Zusatz eines mit Wasser mischbaren organischen Lösungsmittels und/oder vorzugsweise eines wasserlöslichen Erdalkalisalzes ausgeschieden, abfiltriert und gegebenenfalls umkristallisiert wird.

3. Verfahren nach Anspruch 1, dadurch gekennzeichnet, dass die Abtrennung von Erdalkalisalzen der (6R)-Folinsäure bei annähernd neutralem pH durchgeführt wird.

4. Verfahren nach Anspruch 1, dadurch gekennzeichnet, dass man als Alkalisalz bzw. Erdalkalisalz von (6R,S)-Folinsäure, das Natrium oder insbesondere das Calcium- oder Magnesium-Salz verwendet.

5. Verfahren nach Anspruch 1, dadurch gekennzeichnet, dass man als wasserlösliches Alkali- oder Ammoniumsalz einer anorganischen Säure ein Natrium-, Kalium- oder Ammoniumhalogenid, insbesondere Natriumiodid, Natriumbromid, Natriumchlorid, Kaliumiodid, Kaliumbromid, Kaliumchlorid, Ammoniumiodid, Ammoniumbromid und/oder Ammoniumchlorid verwendet.

6. Verfahren nach mindestens einem der Ansprüche 1 oder 2, dadurch gekennzeichnet, dass man als organisches Lösungsmittel einen niedrigen, wasserlöslichen Alkohol oder ein niedriges, wasserlösliches Keton verwendet.

7. Verfahren nach mindestens einem der Ansprüche 1, 2 oder 6, dadurch gekennzeichnet, dass man als wasserlösliches Erdalkalisalz zur Ausscheidung des (6S)-Folinates ein Calcium- oder Magnesiumhalogenid verwendet.

**Claims**

1. Method for the resolution of (6R,S)-folinates, characterized in that a water-soluble alkali metal, ammonium or alkaline earth metal salt of an inorganic or organic acid is added to an aqueous solution of a salt of (6R,S)-folinic acid, the (6R)-folinate which separates out is filtered off, and the (6S)-folinate is isolated from the filtrate.

2. Method according to Claim 1, characterized in that the (6S)-folinate is separated out of the filtrate by addition of a water-miscible organic solvent and/or, preferably, a water-soluble alkaline earth metal salt, is filtered off and is, where appropriate, recrystallized.

3. Method according to Claim 1, characterized in that the removal of alkaline earth metal salts of (6R)-folinic acid is carried out at an approximately neutral pH.

4. Method according to Claim 1, characterized in that the sodium or, especially, the calcium or magnesium salt is used as alkali metal salt or alkaline earth metal salt of (6R,S)-folinic acid.

5. Method according to Claim 1, characterized in that a sodium, potassium or ammonium halide, in particular sodium iodide, sodium bromide, sodium chloride, potassium iodide, potassium bromide, potassium chloride, ammonium iodide, ammonium bromide and/or ammonium chloride, is used as water-soluble alkali metal or ammonium salt of an inorganic acid.

6. Method according to at least one of Claims 1 or 2, characterized in that a water-soluble lower alcohol or a water-soluble lower ketone is used as organic solvent.

7. Method according to at least one of Claims 1, 2 or 6, characterized in that a calcium or magnesium halide is used as water-soluble alkaline earth metal salt for separating out the (6S)-folinate.

## Revendications

1. Procédé de séparation de (6R,S)-folinates, caractérisé en ce qu'on mélange une solution aqueuse d'un sel de l'acide (6R,S)-folique avec un sel alcalin, d'ammonium ou alcalino-terreux soluble dans l'eau d'un acide inorganique ou organique, en ce qu'on sépare par filtration le (6R)-folinate qui apparaît et en ce qu'on isole du filtrat le (6S)-folinate.

2. Procédé selon la revendication 1, caractérisé en ce qu'on sépare le (6S)-folinate du filtrat par addition d'un solvant organique miscible à l'eau et/ou de préférence d'un sel de métal alcalino-terreux soluble dans l'eau, en ce qu'on filtre et le cas échéant en ce qu'on recristallise.

3. Procédé selon la revendication 1, caractérisé en ce qu'on effectue la séparation de sels alcalino-terreux de l'acide (6R)-folique à pH approximativement neutre.

4. Procédé selon la revendication 1, caractérisé en ce qu'on utilise comme sel de métal alcalin ou alcalino-terreux de l'acide (6R,S)-folique le sel de sodium ou en particulier le sel de calcium ou de magnésium.

5. Procédé selon la revendication 1, caractérisé en ce qu'on utilise comme sel de métal alcalin ou d'ammonium soluble dans l'eau d'un acide inorganique un halogénure de sodium, de potassium ou d'ammonium, en particulier l'iodure de sodium, le bromure de sodium, le chlorure de sodium, l'iodure de potassium, le bromure de potassium, le chlorure de potassium, l'iodure d'ammonium, le bromure d'ammonium et/ou le chlorure d'ammonium.

6. Procédé selon au moins une des revendications 1 ou 2, caractérisé an ce qu'on utilise comme solvant organique un alcool inférieur soluble dans l'eau ou une cétone inférieure soluble dans l'eau.

7. Procédé selon au moins une des revendications 1, 2 ou 6, caractérisé en ce qu'on utilise comme sel de métal alcalin soluble dans l'eau pour la séparation du (6S)-folinate un halogénure de calcium ou de magnésium.